# EUROPÄISCHE PATENTSCHRIFT

(11) **EP 0 885 180 B1**
(45) Veröffentlichungstag und Bekanntmachung des Hinweises auf die Patenterteilung: **11.04.2007**
(21) Anmeldenummer: 97914187.6
(22) Anmeldetag: 04.03.1997
(51) Int. Cl.: C07B 63/04, C07C 231/22, C07C 17/42, C07C 41/46, C07C 67/62, C07D 201/18, C07D 207/267, C07D 223/10

(54) **STABILISIERTE MONOMERENZUSAMMENSETZUNG**
STABILIZED MONOMER COMPOSITION
COMPOSITION MONOMERE STABILISEE

(30) Priorität: 09.03.1996 DE 19609312
(43) Veröffentlichungstag der Anmeldung: 23.12.1998
(73) Patentinhaber: BASF Aktiengesellschaft, 67056 Ludwigshafen (DE)
(72) Erfinder: AUMÜLLER, Alexander, D-67435 Neustadt (DE); KOCH, Andreas, D-67240 Bobenheim-Roxheim (DE); SUTORIS, Heinz, Friedrich, D-67227 Frankenthal (DE); DUPUIS, Jacques, D-67069 Ludwigshafen (DE); NIESSNER, Manfred, D-67105 Schifferstadt (DE)
(86) Internationale Anmeldenummer: PCT/EP1997/001085
(87) Internationale Veröffentlichungsnummer: WO 1997/032833

(56) Entgegenhaltungen:
- EP-A- 0 737 660
- EP-A- 0 791 573
- WO-A-96/29311
- DE-A- 4 328 950
- DE-A- 4 414 773
- DE-A- 19 519 628
- FR-A- 2 084 869
- US-A- 4 670 131

## Beschreibung

Gegenstand der vorliegenden Erfindung sind Monomerenzusammensetzungen, enthaltend
A) Vinylgruppen enthaltende Monomere, in denen an der Vinylgruppe ein Heteroatom aus der Gruppe des Stickstoffs, Schwefels oder Siliciums steht, und
B) mindestens eine N-Oxyl-Verbindung eines sekundären Amins, welches keine Wasserstoffatome an den α-C-Atomen trägt, in einer als Stabilisator gegen vorzeitige Polymerisation wirksamen Menge.

Weiterhin betrifft die Erfindung Verfahren zur Inhibierung der vorzeitigen Polymerisation von Vinylgruppen enthaltenden Monomeren sowie die Verwendung von N-Oxyl-Verbindungen von sekundären Aminen, welche keine Wasserstoffatome an den α-C-Atomen tragen, als Stabilisatoren gegen vorzeitige Polymerisation.

Zur Verhinderung vorzeitiger Polymerisation ist es erforderlich, den Monomeren Stabilisatoren zuzusetzen. Als besonders geeignet zur Verhinderung radikalischer Polymerisationen haben sich sterisch gehinderte Amine wie 2,2,6,6-Tetraalkylpiperidin und deren Derivate, darunter auch die N-Oxylderivate, erwiesen.

In US-A 5 254 760 wird die Stabilisierung von vinylaromatischen Verbindungen wie Styrol während der Destillation und Reinigung durch eine Kombination von mindestens einer Nitroxylverbindung und mindestens einer aromatischen Nitroverbindung beschrieben. Dabei besteht die Gefahr, daß Spuren der Nitroxylverbindungen in das gereinigte Monomere gelangen. Schon Spuren von Nitroxylverbindungen stören jedoch die anschließende Polymerisation; sie bewirken eine verzögerte Polymerisation und unkontrollierte Kettenabbrüche, was zu Polymeren mangelhafter Reproduzierbarkeit und kurzer Kettenlänge führt. Diese nachteiligen Effekte werden von Mardare et al in Polym. Prep. (Am. Chem. Soc., Div. Polym. Sci.) 35 (1), 778 (1994) beschrieben.

Zur Stabilisierung von heterosubstituierten Vinylverbindungen wie N-Vinylformamid oder N-Vinylpyrrolidon wurden bei der Destillation und Reinigung bisher beispielsweise Derivate des Phenylendiamins (US-5 396 005), Fullerene (DE-A 44 14 773) oder 2,6-Di-tert.-butyl-p-kresol (DE-A 43 28 950) eingesetzt. Diese Stabilisatoren lassen jedoch hinsichtlich ihrer Wirksamkeit noch zu wünschen übrig.

Aufgabe der vorliegenden Erfindung war es daher, Monomerenzusammensetzungen zu finden, welche heterosubstituierte Vinylverbindungen und geeignete Stabilisatoren enthalten, die eine verbesserte Stabilisierung gegen vorzeitige Polymerisation zeigen, und die sich auf die nachfolgende gezielte Polymerisation der Monomeren kaum nachteilig auswirken.

Demgemäß wurden die eingangs genannten Monomerenzusammensetzungen gefunden.

Die heterosubstituierten Vinylmonomere tragen als Heteroatom an der Vinylgruppe vorzugsweise Stickstoff oder Schwefel.

Als heterosubstituierte Vinylmonomere kommen beispielsweise Vinylthioether, Vinylcarbazole, Vinylpyrrolidone, Vinylphthalimide, Vinylisocyanate, Vinylcaprolactame, Vinylimidazole, Vinylformamid, Vinylsulfonsäure und Vinylsilane wie Vinyltriacetoxysilan, Vinyltrichlorosilan oder Vinyltrimethoxysilan in Betracht.

Bevorzugte Monomerenzusammensetzungen enthalten
A) Monomere der allgemeinen Formel I

   CH₂ = CH - X - R¹ I,

   wobei
   - X: eine Gruppe -NR²-,
   - R¹: 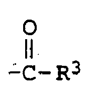 oder -R³,
   - R²: Wasserstoff, C₁-C₄-Alkyl oder gemeinsam mit R³ eine gesättigte oder ungesättigte C₃-, C₄- oder C₅-Alkylenbrücke, in der bis zu zwei Gruppen CH₂ durch NH, N(C₁-C₄-Alkyl), N(C₆-C₁₀-Aryl) oder Sauerstoff und bis zu zwei Gruppen CH durch N ersetzt sein können und
   - R³: Wasserstoff, C₁-C₄-Alkyl oder einen Rest, der zusammen mit R² eine gesättigte oder ungesättigte C₃-, C₄- oder C₅-Alkylenbrücke, in der bis zu zwei Gruppen CH₂ durch NH, N(C₁-C₄-Alkyl), N(C₆-C₁₀-Aryl) oder Sauerstoff und bis zu zwei Gruppen CH durch N ersetzt sein können bedeuten, und
B) mindestens eine N-oxyl-Verbindung eines sekundären Amins, welches keine Wasserstoffatome an den α-C-Atomen trägt, in einer als Stabilisator gegen vorzeitige Polymerisation wirksamen Menge.

Ist die Variable X eine Gruppe -NR²-, so ist R¹ vorzugsweise eine Gruppe -CO-R³.

Als Reste R³ kommen neben Wasserstoff und den genannten C₁-C₄-Alkylgruppen auch solche Reste in Betracht, die zusammen mit der Gruppe -NR²- einen gesättigten oder ungesättigten 5- bis 7-gliedrigen Ring bilden. Beispiele solcher Ringsysteme sind: 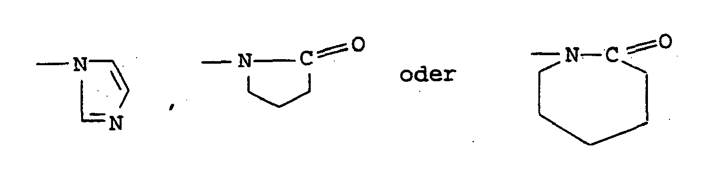 darunter besonders der N-Pyrrolidinonyl- und der N-Caprolactamylrest.

Bevorzugte Monomere in den erfindungsgemäßen Zusammensetzungen sind N-Vinylformamid, N-Vinyl-2-pyrrolidon oder N-Vinyl-ε-caprolactam,

Besonders bevorzugt unter diesen Monomeren ist N-Vinylformamid.

Als Stabilisatoren (B) enthalten die erfindungsgemäßen Monomerenzusammensetzungen mindestens eine N-Oxyl-Verbindung eines sekundären Amins, welches keine Wasserstoffatome an den α-C-Atomen trägt. Diese Verbindungen können als freie Verbindungen oder in Form ihrer Salze vorliegen.

Geeignete N-Oxyle von Aminen sind z.B. die folgenden Strukturen 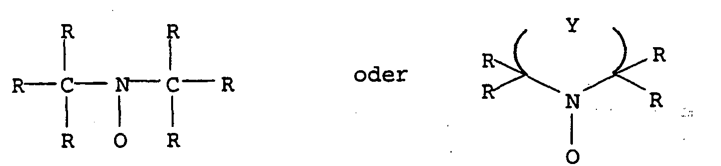 wobei R gleiche oder verschiedene Alkyl-, Cycloalkyl-, Aralkyl- oder Arylreste, die auch paarweise zu einem Ringsystem verbunden sein können, und Y eine Gruppe, die erforderlich ist, um einen 5-oder 6-gliedrigen Ring zu vervollständigen, bedeuten. Beispielsweise steht R für einen C₁-C₂₀-, insbesondere C₁-C₈-Alkylrest, einen C₅- oder C₆-cycloalkylrest, einen Benzylrest oder einen Phenylrest. Y ist beispielsweise eine Alkylengruppe -(CH2)2- oder -(CH₂)₃-.

Weiterhin kommen auch N-Oxylverbindungen wie die folgenden Strukturen in Betracht 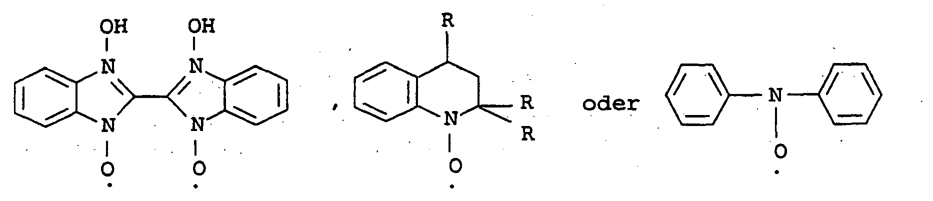 wobei die aromatischen Ringe jeweils noch 1 bis 3 inerte Substituenten tragen können, wie z.B. C₁-C₄-Alkyl, C₁-C₄-Alkoxy oder Cyano.

Vorzugsweise werden sterisch gerinderte Aminderivate von cyclischen Aminen eingesetzt, z.B. von Piperidin- oder Pyrrolidinverbindungen, die im Ring ein weiteres Heteroatom wie Stickstoff, Sauerstoff oder Schwefel enthalten können, wobei dieses Heteroatom nicht in Nachbarstellung zum gehinderten Aminstickstoff steht. Die sterische Hinderung ist durch Substituenten in beiden Nachbarstellungen zum Aminstickstoff gegeben, wobei als Substituenten Kohlenwasserstoffreste in Betracht kommen, die alle 4 Wasserstoffatome der α-CH₂-Gruppen ersetzen. Beispielsweise seien als Substituenten Phenyl, C₃-C₆-Cycloalkyl, Benzyl und insbesondere C₁-C₆-Alkylreste genannt, wobei die an dem selben α-C-Atom gebundenen Alkylreste auch untereinander zu einem 5-oder 6-Ring verbunden sein können. Besonders bevorzugt sind die unter R⁴,R⁵ im einzelnen aufgeführten Reste. Vorzugsweise werden als N-Oxyle sterisch gehinderter Amine Derivate des 2,2,6,6-Tetraalkylpiperidins eingesetzt.

Bevorzugte N-Oxyl-Verbindungen in den erfindungsgemäßen Monomerenzusammensetzungen sind solche der allgemeinen Formel II 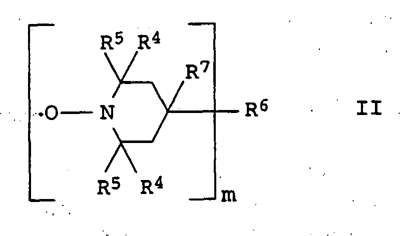 wobei
- R⁴, R⁵: C₁-C₄-Alkyl, Phenyl oder gemeinsam mit dem C-Atom, an das sie gebunden sind; einen 5- oder 6-gliedrigen gesättigten Kohlenwasserstoffring,
- R⁶: Wasserstoff, Hydroxyl, Amino oder einen m-wertigen über Sauerstoff oder Stickstoff gebundenen organischen Rest oder zusammen mit R⁷ Sauerstoff oder eine unter R⁷ definierte Ringstruktur,
- R⁷: Wasserstoff, C₁-C₁₂-Alkyl oder zusammen mit R⁶ Sauerstoff oder zusammen mit R⁶ und dem C-Atom, an das sie gebunden sind, folgende Ringstrukturen 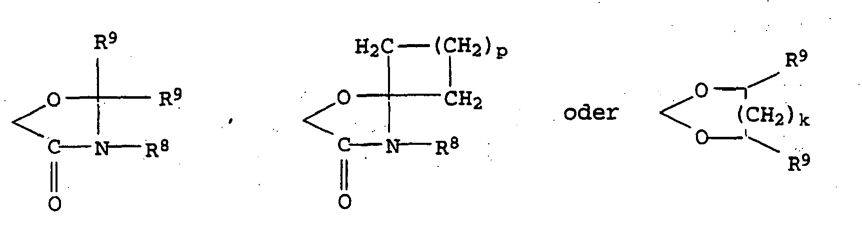 wobei für die Fälle, in denen R⁶ mit R⁷ einen gemeinsamen Rest bildet, m = 1 ist,
- R⁸: Wasserstoff, C₁-C₁₂-Alkyl oder -(CH₂)_{z}-COOR⁹,
- R⁹: gleiches oder verschiedenes C₁-C₁₈-Alkyl,
- k: 0 oder 1
- z, p: 1 bis 12 und
- m: 1 bis 100
bedeuten.

R⁴ und R⁵ können z.B. die für R¹ genannten C₁-C₄-Alkylgruppen sein oder sie können zusammen eine Tetra- oder Pentamethylengruppe bilden. Bevorzugt sind R⁴ und R⁵ Methylgruppen.

Als R⁷ kommen beispielsweise Wasserstoff, die oben genannten C₁-C₄-Alkylgruppen sowie Pentyl, sec-Pentyl, tert-Pentyl, Neopentyl, Hexyl, 2-Methylpentyl, Heptyl, 2-Methylhexyl, Octyl, Isooctyl, 2-Ethylhexyl, Nonyl, 2-Methylnonyl, Isononyl, 2-Methyloctyl, Decyl, Isodecyl, 2-Methylnonyl, Undecyl, Isoundecyl, Dodecyl und Isododecyl, (die Bezeichnungen Isooctyl, Isononyl und Isodecyl sind Trivialbezeichnungen und stammen von den nach der Oxosynthese erhaltenen Carbonylverbindungen ab; vgl. dazu Ullmann's Encyklopedia of Industrial Chemistry, 5th Edition, Vol. A1. Seiten 290-293, sowie Vol. A10, Seiten 284 und 285) in Betracht.
- p: ist bevorzugt 6-12, besonders bevorzugt 9.
- z: ist bevorzugt 1-4, besonders bevorzugt 2.

Als R⁸ kommen neben Wasserstoff beispielsweise die oben angegebenen C₁-C₁₂-Alkylgruppen in Betracht. Bevorzugt steht R⁸ für Wasserstoff, C₁-C₄-Alkyl oder (CH₂)_{z}-COO(C₁-C₆-Alkyl), besonders bevorzugt für die Reste -CH₂-CH₂-COO(CH₂)₁₁-CH₃ und -CH₂-CH₂-COO(CH₂)₁₃-CH₃·

R⁹ kann beispielsweise eine der oben genannten C₁-C₁₂-Alkylgruppen oder Tridecyl, Isotridecyl, Tetradecyl, Pentadecyl, Hexadecyl, Heptadecyl oder Octadecyl sein. Bevorzugt sind Dodecyl und Hexadecyl.

Bevorzugte Reste R⁶ sind beispielsweise die folgenden m-wertigen Reste 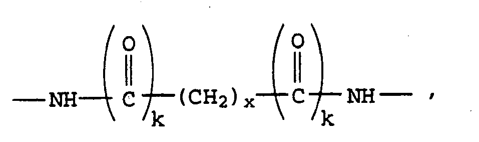 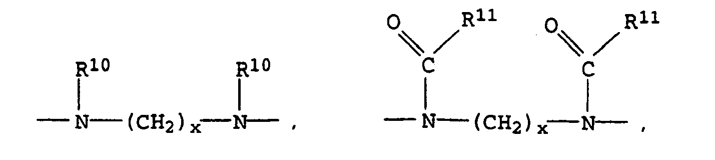 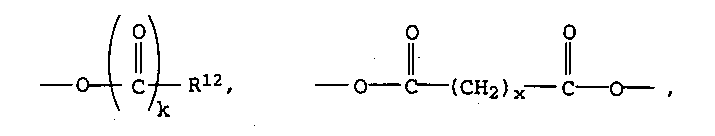 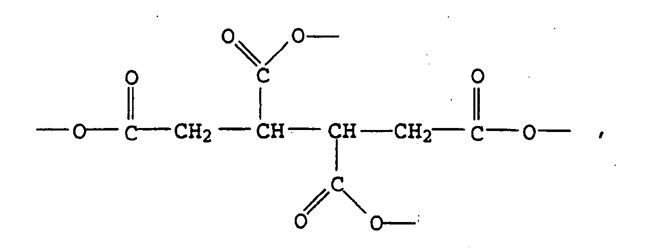 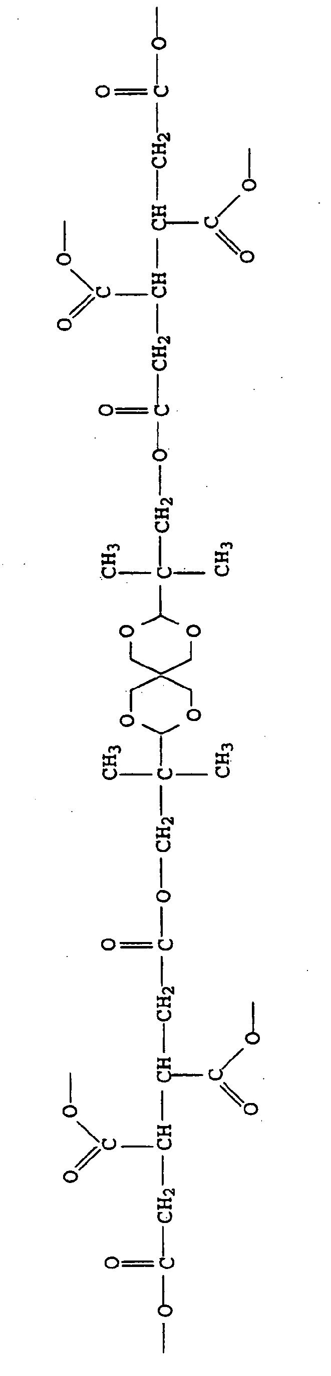 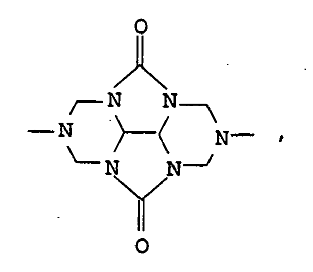 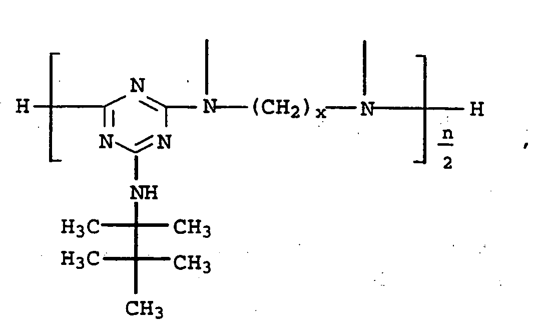 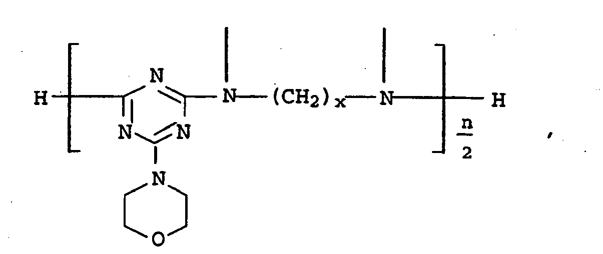 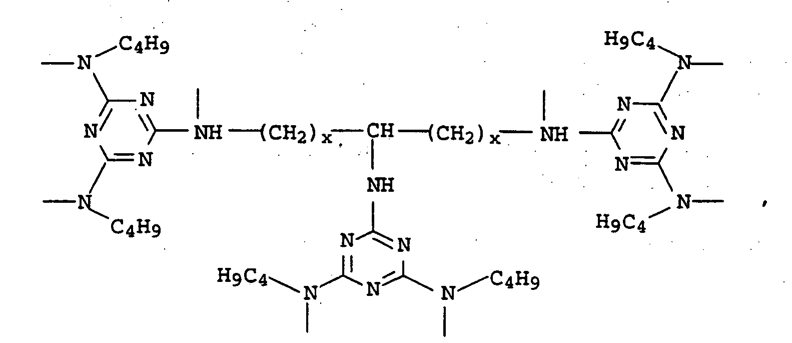 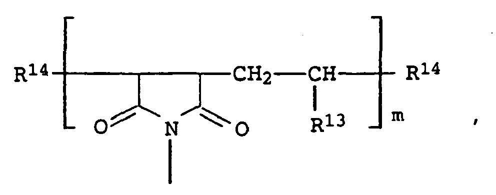 wobei
- R¹⁰: C₁-C₁₂-Alkyl oder - (CH₂)_{z}-COOR⁹
- R¹¹: Wasserstoff oder C₁-C₁₈-Alkyl,
- R¹²: C₁-C₁₈-Alkyl, Vinyl oder Isopropenyl,
- R¹³: C₈-C₂₂-Alkyl,
- R¹⁴: Wasserstoff oder einen organischen Rest, wie er bei der radikalischen Polymerisation der Ausgangsmonomeren üblicherweise entsteht,
- k: 0 oder 1,
- x: 1 bis 12 und
- n: eine gerade Zahl m
bedeuten.

Ist R⁶ einer dieser Reste, so ist R⁷ bevorzugt Wasserstoff. Die Variable m kann dabei 1 bis 100 bedeuten. Bevorzugt ist m 1,2,3,4 oder eine Zahl von 10 bis 50, wobei besonders bei den oligomeren oder polymeren Resten R⁶ in der Regel Gemische eingesetzt werden.

Als R¹⁰ kommen die gleichen Reste in Betracht, wie sie für R⁸ genannt sind. Bevorzugt steht R¹⁰ für C₁-C₄-Alkyl.

Als R¹¹ kommen neben Wasserstoff die gleichen Reste in Betracht, wie sie für R⁹ genannt worden sind. Bevorzugt steht R¹¹ für Wasserstoff.

Als R¹² kommen besonders Vinyl, Isopropenyl oder C₁₅-C₁₇-Alkylreste in Betracht.

Als R¹³ kommen beispielsweise die oben genannten C₈-C₁₈-Alkylreste sowie Nonadecyl, Eicosyl, Uneicosyl und Doeicosyl in Betracht. Dabei sind Mischungen verschiedener Reste R¹³, die sich in der Länge der Kohlenstoffkette unterscheiden, bevorzugt.

Die Reste R¹⁴ sind Wasserstoff oder organische Reste, wie sie bei der radikalischen Polymerisation der Ausgangsmonomeren, in diesem Fall von einem Ethylenderivat und einem Maleinsäureimidderivat, entstehen, also z.B. ein Rest, der aus dem Polymerisationsinitiator oder aus einem intermediär aufgetretenen Radikal entsteht oder ein anderer derartiger Rest, wie er dem Fachmann geläufig ist.

Bevorzugte Nitroxylverbindungen als Komponente B) der erfindungsgemäßen Monomerenzusammensetzungen sind auch die folgenden :
1-Oxyl-2,2,6,6-tetramethylpiperidin,
1-Oxyl-2,2,6,6-tetramethylpiperidin-4-ol,
1-Oxyl-2,2,6 6-tetramethylpipäridin-4-on,
1-Oxyl-2,2,6,6-tetramethylpiperidin-4-yl-acetat,
1-Oxyl-2,2,6,6-tetramethylpiperidin-4-yl-2-ethylhexanoat,
1-Oxyl-2,2,6,6-tetramethylpiperidin-4-yl-stearat,
1-Oxyl-2,2,6,6-tetramethylpiperidin-4-yl-benzoat,
1-Oxyl-2,2,6,6-tetramethylpiperidin-4-yl-(4-tert-butyl)benzoat,
Bis(1-oxyl-2,2,6,6-tetramethylpiperidin-4-yl)-succinat,
Bis(1-oxyl-2,2,6,6-tetramethylpiperidin-4-yl)-adipat,
Bis(1-oxyl-2,2,6,6-tetramethylpiperidin-4-yl)-sebacat,
Bis (1-oxyl-2,2,6,6-tetramethylpiperidin-4-yl)-n-butylmalonat,
Bis(1-oxyl-2,2,6,6-tetramethylpiperidin=4-yl)-phthalat,
Bis(1-oxyl-2,2,6,6-tetramethylpiperidin-4-yl)-isophthalat,
Bis(1-oxyl-2,2,6,6-tetramethylpiperidin-4-yl)-terephthalat,
Bis(1-oxyl-2,2,6,6-tetramethylpiperidin-4-yl)-hexyhydroterephthalat,
N,N'-Bis(1-oxyl-2,2,6,6-tetramethylpiperidin-4-yl)-adipinamid,
N-(1-Oxyl-2,2,6,6-tetramethylpiperidin-4-yl)-caprolactam,
N-(1-Oxyl-2,2,6,6-tetramethylpiperidin-4-yl)-dodecylsuccinimid,
2,4,6-Tris-[N-butyl-N-(1-oxyl-2,2,6,6,-tetramethylpiperidin-4-yl]-s-triazin,
4,4'-Ethylenbis(1-oxyl-2,2,6,6-tetramethylpiperazin-3-on) und
Tris-(2,2,6,6-tetramethyl-1-oxyl-piperidin-4-yl)phosphit:

Die beschriebenen Nitroxylverbindungen lassen sich aus den entsprechenden Piperidinverbindungen durch Oxidation z.B. mit Wasserstoffperoxid herstellen. Details zu dieser Oxidation sind z.B. in der älteren deutschen Patentanmeldung 195 101 84.7 genannt. Die sekundären Amine, welche an den α-C-Atomen keine Wasserstoffatome tragen, wie Piperidinverbindungen, und ihre Herstellung sind allgemein bekannt. Da die Oxidationsreaktionen nicht immer vollständig ablaufen, können auch die als Ausgangsverbindungen dienenden Piperidinverbindungen sowie teilweise oxidierte Zwischenstufen in den erfindungsgemäßen Monomerenzusammensetzungen enthalten sein.

Besonders geeignete Monomerenzusammensetzungen sind solche, die neben den erwähnten Nitroxylverbindungen zur Stabilisierung zusätzlich eine oder mehrere aromatische Nitroso- oder Nitroverbindungen enthalten.

Als aromatische Nitroverbindungen können beispielsweise 1,3-Dinitrobenzol, 1,4-Dinitrobenzol, 2,6-Dinitro-4-methylphenol, 2-Nitro-4-methylphenol, 2,4,6-Trinitrophenol, 2,4-Dinitro-1-naphthol, 2,4-Dinitro-6-methylphenol, 2,4-Dinitrochlorbenzol, 2,4-Dinitrophenol, 2,4-Dinitro-6-sec-butylphenol, 4-Cyano-2-nitrophenol, 3-Iodo-4-cyano-5-nitrophenol, besonders bevorzugt 2,6-Dinitro-4-methylphenol, 2-Nitro-4-methylphenol, 2,4-Di-nitro-6-sec-butylphenol bzw. 2,4-Dinitro-6-methylphenol verwendet werden.

Als aromatische Nitrosoverbindungen kommen z.B. p-Nitrosophenol, p-Nitroso-o-kresol und p-Nitroso-N,N'-diethylanilin in Betracht.

Weiterhin können den Monomerenzusammensetzungen als Costabilisatoren auch substituierte Phenole zugesetzt werden, wie beispielsweise die folgenden:
4-tert-Butylbrenzcatechin, Methoxyhydrochinon, 2,6-Di-tert-butyl-4-methylphenol, n-Octadecyl-β-(3,5-di-tert-butyl-4-hydroxyphenyl)-propionat, 1,1,3-Tris-(2-methyl-4-hydroxy-5-tert-butylphenyl)-butan, 1,3,5-Trimethyl-2,4,6-tris-(3,5-di-tert-butyl-4-hydroxybenzyl)-benzol, 1,3,5-Tris-(3,5-di-tert-butyl-4-hydroxybenzyl)-isocyanurat, 1,3,5-Tris-[β-(3,5-di-tert-butyl-4-hydroxyphenyl)-propionyloxyethyl-isocyanurat, 1,3,5-Tris-(2,6-dimethyl-3-hydroxy-4-tert-butylbenzyl)-isocyanurat und Pentaerythrit-tetrakis-[β-(3,5-di-tert-butyl-4-hydroxy-phenyl)-propionat].

Weiterhin können die erfindungsgemäßen Mononierenzusammensetzungen auch einen oder mehrere Costabilisatoren aus der Gruppe der Phenothiazine, Chinone, Hydroxylamine oder Phenylendiamine enthalten.

Zur Stabilisierung der erfindungsgemäßen Monomerenzusammensetzungen enthalten diese Zusammensetzungen im allgemeinen 0,0002 bis 5, vorzugsweise 0,0005 bis 0,5 Gew.-% der Nitroxylverbindungen, bezogen auf die Gesamtmenge der Monomerenzusammensetzung.

Die Stabilisatoren zeigen ihre stabilisierende Wirkung in einem breiten Temperaturbereich. Sie sind bei jeder üblichen Lagertemperatur von -50 bis +50°C wirksam und ebenso bei erhöhten Temperaturen, wie sie beispielsweise bei der Destillation der Monomeren angewendet werden. Auch der Druckbereich des Stabilisierungsverfahrens ist unkritisch. Die Stabilisatoren wirken bei Normaldruck und auch bei vermindertem Druck wie er teilweise bei Destillationsprozessen angewendet wird.

Das erfindungsgemäße Verfahren zur Inhibierung der vorzeitigen Polymerisation von Monomeren findet seinen Einsatz während der Herstellung, der Destillation oder Reinigung der Monomeren sowie auch bei ihrer Lagerung und beim Transport.

Lagerung von N-Vinylformamid in Gegenwart von N,N'-Bis(1-oxyl-2,2,6,6-tetramethylpiperidin-4-yl)-N,N' bis

Eine Monomerenzusammensetzung aus Vinylformamid und 0,05 Gew.-% N,N'-Bis(1-oxyl-2,2,6,6-tetramethylpiperidin-4-yl)-N,N'- bis-formyl-1,6-diaminohexan, bezogen auf die Gesamtmenge der Zusammensetzung, wurde bei 40°C gelagert. Nach der in der Tabelle angegebenen Zeiten wurde eine Probe entnommen une der Gehalt an Vinylformamid durch iodometrische Titration bestimmt. In der Tabelle sind als Maß für den jeweiligen Gehalt an Vinylformamid die

| Lagerzeit [Tage] | Iodzahl ohne Stabilisator | Iodzahl mit Stabilisator |
|---|---|---|
| 0 | 98,8 | 98,2 |
| 20 | 89,9 | 92,8 |
| 41 | 80,5 | 87,5 |
| 62 | 71.5 | 81.2 |
| 83 | 62,5 | 75,7 |

## Patentansprüche

1. Monomerenzusammensetzung enthaltend
A) Vinylgruppen enthaltende Monomere, in denen an der Vinylgruppe ein Heteroatom aus der Gruppe des Stickstoffs, Schwefels oder Siliciums steht, und
B) mindestens eine N-Oxyl-Verbindung eines sekundären Amins, welches keine Wasserstoffatome an den α-C-Atomen trägt, in einer als Stabilisator gegen vorzeitige Polymerisation wirksamen Menge.

2. Monomerenzusammensetzung nach Anspruch 1, enthaltend
A) Monomere der allgemeinen Formel I
CH₂ = CH - X - R¹ I,
wobei
X eine Gruppe -NR²-,
R¹ 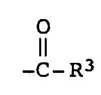 oder -R³,
R² Wasserstoff, C₁-C₄-Alkyl oder gemeinsam mit R³ eine gesättigte oder ungesättigte C₃-, C₄- oder C₅-Alkylenbrücke, in der bis zu zwei Gruppen CH₂ durch NH, N(C₁-C₄-Alkyl), N(C₆-C₁₀-Aryl) oder Sauerstoff und bis zu zwei Gruppen CH durch N ersetzt sein können und
R³ Wasserstoff, C₁-C₄-Alkyl oder einen Rest, der zusammen mit R² eine gesättigte oder ungesättigte C₃-, C₄- oder C₅-Alkylenbrücke, in der bis zu zwei Gruppen CH₂ durch NH, N(C₁-C₄-Alkyl), N(C₆-C₁₀-Aryl) oder Sauerstoff und bis zu zwei Gruppen CH durch N ersetzt sein können
bedeuten, und
B) mindestens eine N-oxyl-Verbindung eines sekundären Amins, welches keine Wasserstoffatome an den α-C-Atomen trägt, in einer als Stabilisator gegen vorzeitige Polymerisation wirksamen Menge.

3. Monomerenzusammensetzung nach Anspruch 1 oder 2, enthaltend als Monomere N-Vinylformamid, N-Vinylpyrrolidon oder N-Vinylcaprolactam.

4. Monomerenzusammensetzung nach Anspruch 1 oder 2, enthaltend als Monomeres N-Vinylformamid.

5. Monomerenzusammensetzung nach den Ansprüchen 1 bis 4, die als Stabilisator eine Verbindung oder eine Mischung von Verbindungen der allgemeinen Formel II 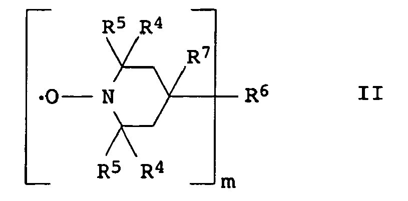 enthalten, wobei
R⁴, R⁵ C₁-C₄-Alkyl, Phenyl oder gemeinsam mit dem C-Atom, an das sie gebunden sind, einen 5- oder 6-gliedrigen gesättigten Kohlenwasserstoffring,
R⁶ Wasserstoff, Hydroxyl, Amino oder einen m-wertigen über Sauerstoff oder Stickstoff gebundenen organischen Rest oder zusammen mit R⁷ Sauerstoff oder eine unter R⁷ definierte Ringstruktur,
R⁷ Wasserstoff, C₁-C₁₂-Alkyl oder zusammen mit R⁶ Sauerstoff oder zusammen mit R⁶ und dem C-Atom, an das sie gebunden sind, folgende Ringstrukturen 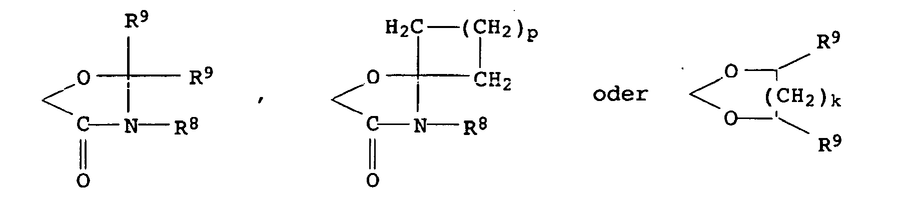 wobei für die Fälle, in denen R⁶ mit R⁷ einen gemeinsamen Rest bildet, m = 1 ist,
R⁸ Wasserstoff, C₁-C₁₂-Alkyl oder -(CH₂)_{z}-COOR⁹,
R⁹ gleiches oder verschiedenes C₁-C₁₈-Alkyl,
k 0 oder 1
z, p 1 bis 12 und
m 1 bis 100
bedeuten.

6. Monomerenzusammensetzung nach Anspruch 5, wobei R⁶ in Formel II einen Rest aus der folgenden Gruppe bedeutet: 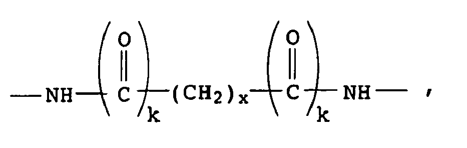 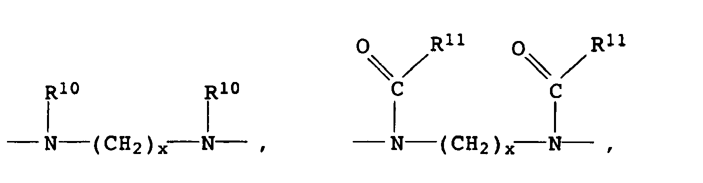 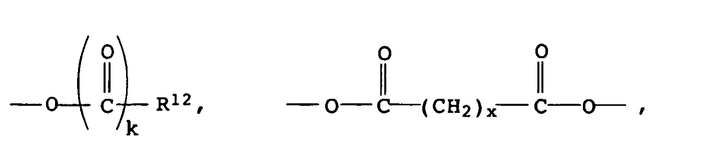 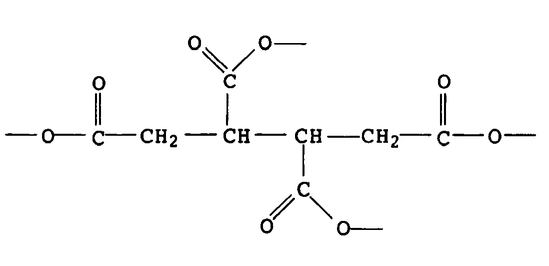 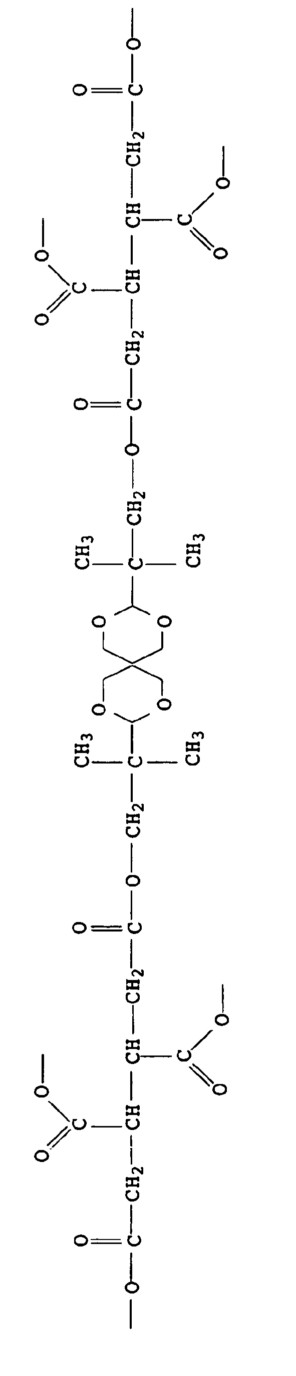 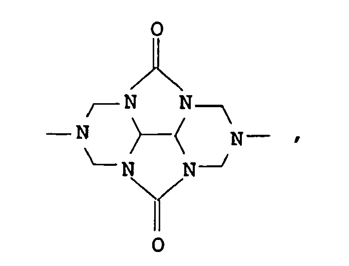 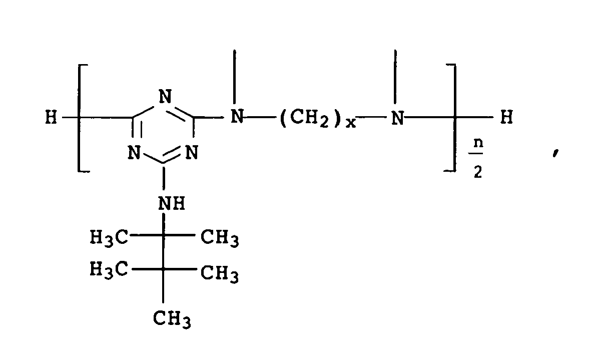 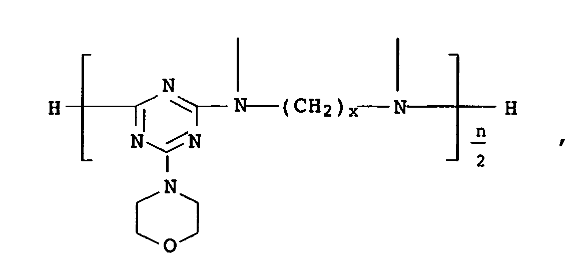 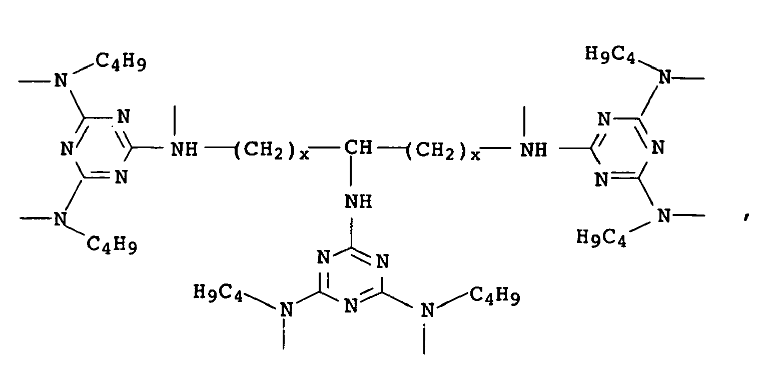 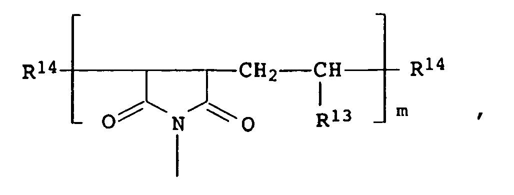 wobei
R¹⁰ C₁-C₁₂-Alkyl oder -(CH₂)_{z}-COOR⁹
R¹¹ Wasserstoff oder C₁-C₁₈-Alkyl,
R¹² C₁-C₁₈-Alkyl, Vinyl oder Isopropenyl,
R¹³ C₈-C₂₂-Alkyl,
R¹⁴ Wasserstoff oder einen organischen Rest, wie er bei der radikalischen Polymerisation der Ausgangsmonomeren üblicherweise entsteht,
k 0 oder 1,
x 1 bis 12 und
n eine gerade Zahl m
bedeuten.

7. Monomerenzusammensetzung nach den Ansprüchen 1 bis 6, welche zusätzlich eine oder mehrere aromatische Nitroso- oder Nitroverbindungen enthalten.

8. Monomerenzusammensetzung nach den Ansprüchen 1 bis 6, welche zusätzlich einen oder mehrere Costabilisatoren aus der Gruppe der Phenothiazine, Chinone, Hydrochinone und deren Ether, Hydroxylamine oder Phenylendiamine enthalten.

9. Verfahren zur Inhibierung der vorzeitigen Polymerisation von Monomeren gemäß Anspruch 1, **dadurch gekennzeichnet, daß** man den Monomeren gemäß Anspruch 1 mindestens eine N-Oxylverbindung eines sekundären Amins, welches keine Wasserstoffatome an den α-C-Atomen trägt, in einer als Stabilisator wirksamen Menge zusetzt.

10. Verfahren nach Anspruch 9, **dadurch gekennzeichnet, daß** man den Monomeren mindestens eine Tetraalkylpiperidin-N-oxylverbindung der Formel II gemäß den Ansprüchen 5 und 6 zusetzt.

11. Verfahren nach den Ansprüchen 9 und 10 **dadurch gekennzeichnet, daß** das zu stabilisierende Monomere aus der Gruppe N-Vinylformamid, N-Vinylpyrrolidon und N-Vinylcaprolactam stammt.

12. Verwendung von Verbindungen der Formel II gemäß den Ansprüchen 5 und 6 zur Stabilisierung von Monomeren der Formel I gemäß Anspruch 2 gegen vorzeitige Polymerisation.

## Claims

1. A monomer composition comprising
A) vinyl-comprising monomers in which the vinyl bears a heteroatom selected from the group consisting of nitrogen, sulfur or silicon, and
B) at least one N-oxyl compound of a secondary amine which does not bear hydrogen on the α-carbons, in an amount effective as stabilizer against premature polymerization.

2. The monomer composition according to claim 1, comprising
A) monomers of the general formula I
CH₂ = CH - X - R¹ I,
where
X is -NR²-,
R¹ is 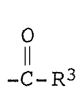 or -R³
R² is hydrogen, C₁-C₄-alkyl or, together with R³, is a saturated or unsaturated C₃-, C₄- or C₅-alkylene bridge in which up to two CH₂ groups can be replaced by NH, N(C₁-C₄-alkyl), N(C₆-C₁₀-aryl) or oxygen and up to two CH groups can be replaced by N and
R³ is hydrogen, C₁-C₄-alkyl or a radical which, together with R², is a saturated or unsaturated C₃-, C₄- or C₅-alkylene bridge in which up to two CH₂ groups can be replaced by NH, N(C₁-C₄-alkyl), N(C₆-C₁₀-aryl) or oxygen and up to two CH groups can be replaced by N,
and
B) at least one N-oxyl compound of a secondary amine which does not bear hydrogen on the α-carbons in an amount effective as stabilizer against premature polymerization.

3. The monomer composition according to claim 1 or 2, comprising as monomers N-vinylformamide, N-vinylpyrrolidone or N-vinylcaprolactam.

4. The monomer composition according to claim 1 or 2, comprising as monomer N-vinylformamide.

5. The monomer composition according to any of claims 1 to 4, which comprise as stabilizer a compound or a mixture of compounds of the general formula II 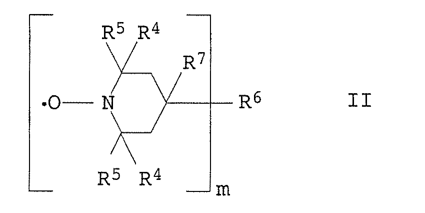 where
R⁴, R⁵ are C₁-C₄-alkyl, phenyl or, together with the carbon to which they are bound, are a 5- or 6-membered saturated hydrocarbon ring,
R⁶ is hydrogen, hydroxyl, amino or an m-valent organic radical bound by oxygen or nitrogen or, together with R⁷, is oxygen or a ring structure defined under R⁷,
R⁷ is hydrogen, C₁-C₁₂-alkyl or, together with R⁶, is oxygen or, together with R⁶ and the carbon to which they are bound, is the following ring structures 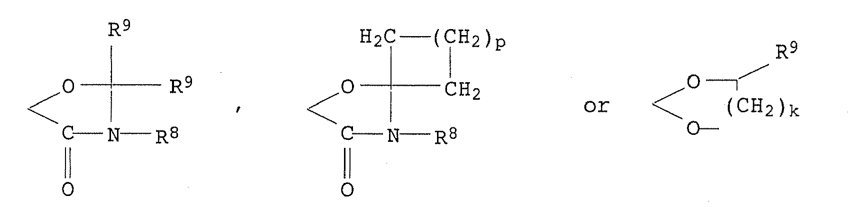 where, for the cases when R⁶ jointly forms a radical with R⁷, m = 1,
R⁸ is hydrogen, C₁-C₁₂-alkyl or -(CH₂)_{z}-COOR⁹,
R⁹ is identical or different C₁-C₁₈-alkyl,
k is 0 or 1
z, p are from 1 to 12 and
m is from 1 to 100.

6. The monomer composition according to claim 5, where R⁶ in formula II is a radical selected from the following group: 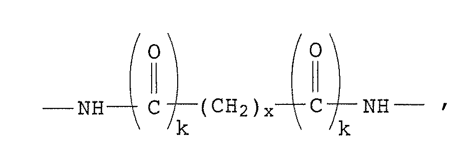 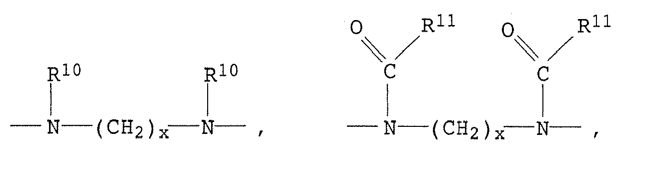 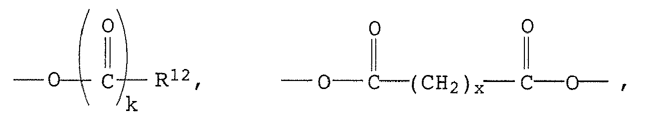 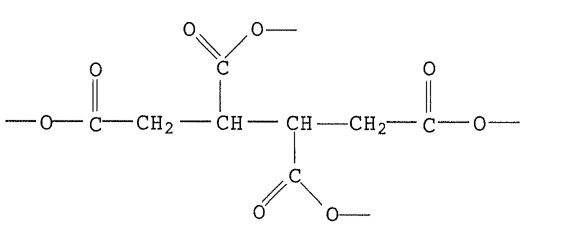 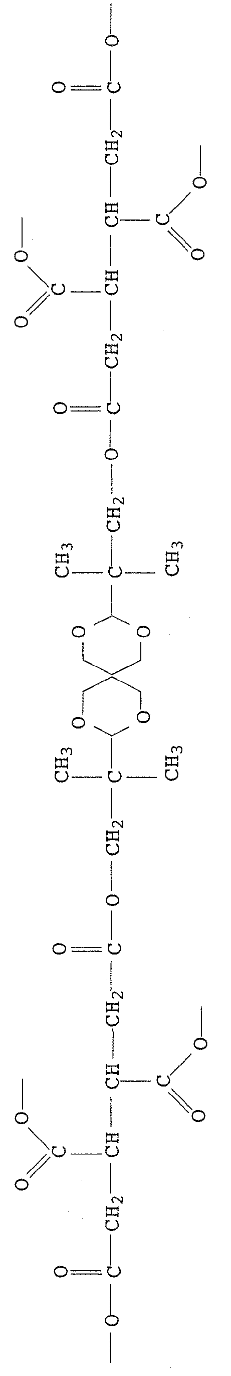 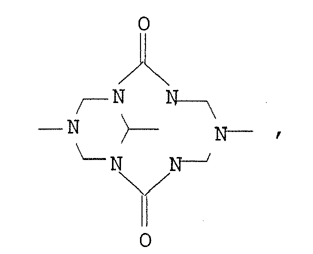 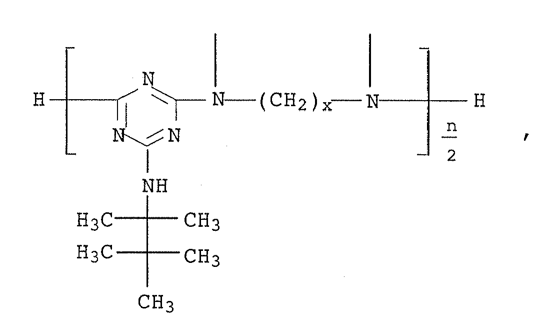 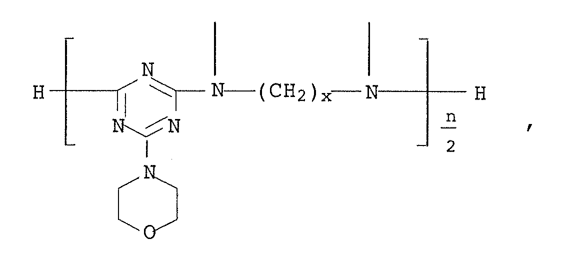 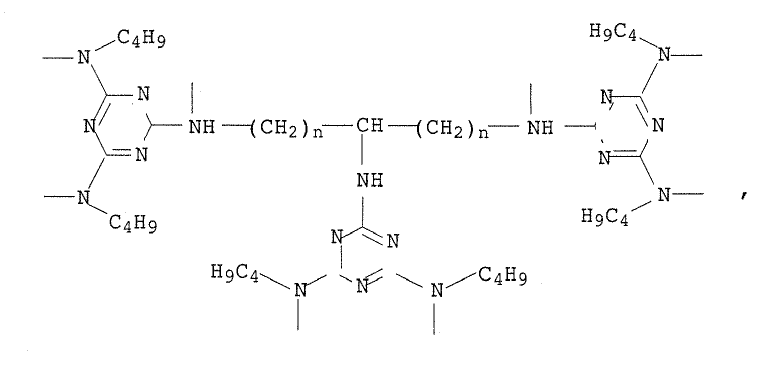 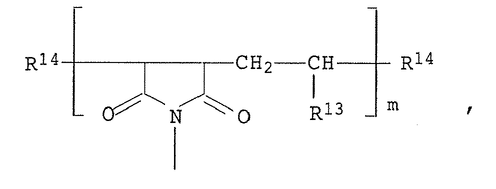 where
R¹⁰ is C₁-C₁₂-alkyl or -(CH₂)_{z}-COOR⁹
R¹¹ is hydrogen or C₁-C₁₈-alkyl,
R¹² is C₁-C₁₈-alkyl, vinyl or isopropenyl,
R¹³ is C₈-C₂₂-alkyl,
R¹⁴ is hydrogen or an organic radical such as is customarily formed in the free-radical polymerization of the starting mono- mers,
k is 0 or 1,
x is from 1 to 12 and
n is an even number m.

7. The monomer composition according to any of claims 1 to 6, which additionally comprises one or more aromatic nitroso or nitro compounds.

8. The monomer composition according to any of claims 1 to 7, which additionally comprises one or more costabilizers selected from the group consisting of phenothiazines, quinones, hydroquinones and their ethers, hydroxylamines or phenylenediamines.

9. A process for inhibiting the premature polymerization of monomers according to claim 1, which comprises adding at least one N-oxyl compound of a secondary amine which does not bear hydrogen on the α-carbons to the monomers according to claim 1, in an amount effective as stabilizer.

10. The process according to claim 9, wherein at least one tetraalkylpiperidine-N-oxyl compound of the formula II according to claims 5 and 6 is added to the monomers.

11. The process according to claims 9 and 10, wherein the monomer to be stabilized comes from the groups consisting of N-vinylformamide, N-vinylpyrrolidone and N-vinylcaprolactam.

12. The use of compounds of the formula II according to claims 5 and 6 for stabilizing monomers of the formula I according to claim 2 against premature polymerization.

## Revendications

1. Composition de monomères contenant
A) des monomères contenant des groupes vinyliques dans lesquels se trouve, sur le groupe vinylique, un hétéroatome du groupe des azote, soufre ou silicium, et
B) au moins un composé N-oxyle d'une amine secondaire, laquelle ne porte aucun atome d'hydrogène sur les atomes de C-α dans une quantité efficace contre une polymérisation précoce comme stabilisant.

2. Composition de monomères selon la revendication 1 contenant
A) des monomères de formule générale I
CH₂ = CH - X- R¹ I
dans laquelle
X est un groupe -NR²-,
R¹ 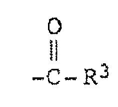 ou -R³,
R² est un hydrogène, un alkyle en C₁-C₄ ou avec R³, un pont alkylène saturé ou insaturé en C₃, C₄ ou C₅, dans lequel deux groupes CH₂ maximum peuvent être remplacés par NH, N(alkyle en C₁-C₄), N(aryle en C₆-C₁₀) ou de l'oxygène et deux groupes CH maximum par du N, et
R³ est un hydrogène, un alkyle en C₁-C₄ ou un radical qui avec R² est un pont alkylène saturé ou insaturé en C₃, C₄ ou C₅, dans lequel deux groupes CH₂ maximum peuvent être remplacés par NH, N (alkyle en C₁-C₄), N(aryle en C₆-C₁₀) ou de l'oxygène et deux groupes CH maximum par du N, et
B) au moins un composé N-oxyle d'une amine secondaire, laquelle ne porte aucun atome d'hydrogène sur les atomes de C-α, dans une quantité efficace contre une polymérisation précoce comme stabilisant.

3. Composition de monomères selon la revendication 1 ou 2, contenant comme monomères du N-vinylformamide, de la N-vinylpyrrolidone ou du N-vinylcaprolactame.

4. Composition de monomères selon la revendication 1 ou 2, contenant comme monomère du N-vinylformamide.

5. Composition de monomères selon les revendications 1 à 4 qui contient comme stabilisant un composé ou un mélange de composés de formule générale II 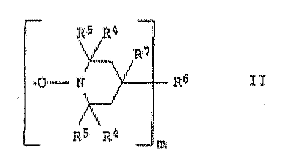 dans laquelle
R⁴, R⁵ signifient un alkyle en C₁-C₄, un phényle ou avec l'atome de carbone, auquel ils sont liés, un cycle hydrocarboné saturé à 5 ou 6 membres,
R⁶ signifie un hydrogène, un hydroxyle, un amino ou un radical organique à valence m lié par de l'oxygène ou de l'azote ou avec R⁷ signifie de l'oxygène ou une structure cyclique définie en R⁷
R⁷ signifie un hydrogène, un alkyle en C₁-C₁₂ ou avec R⁶, un oxygène ou avec R⁶ et l'atome de carbone, auquel ils sont liés, les structures cycliques suivantes 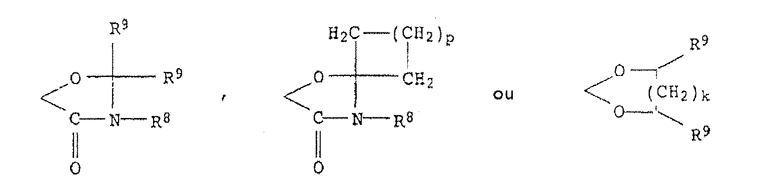 dans lesquelles pour les cas où R⁶ avec R⁷ forme un radical commun, m=1,
R⁸ signifie un hydrogène, un alkyle en C₁-C₁₂ ou - (CH₂)_{z}-COOR⁹
R⁹ signifie, identique ou différent, un alkyle en C₁-C₁₈, k signifie 0 ou 1
z, p signifient 1 à 12, et
m signifie 1 à 100.

6. Composition de monomères selon la revendication 5, dans laquelle R⁶ dans la formule II signifie un radical du groupe suivant: 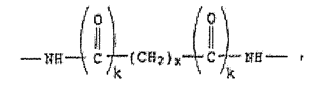 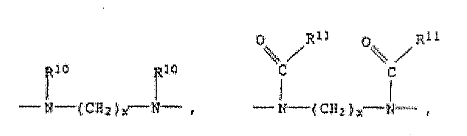 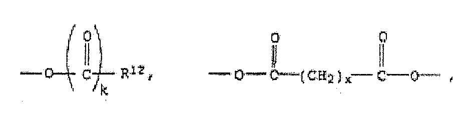 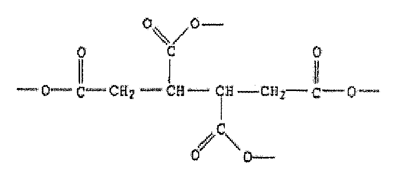 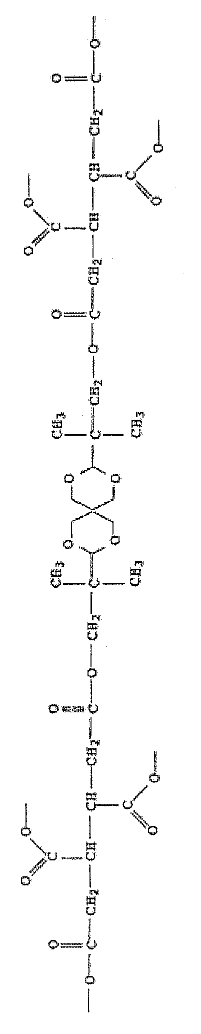 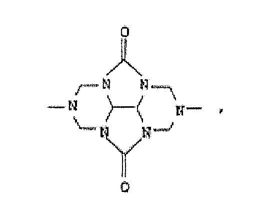 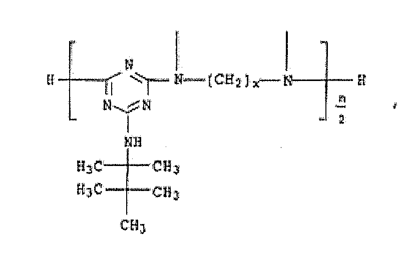 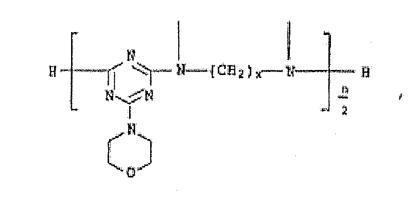 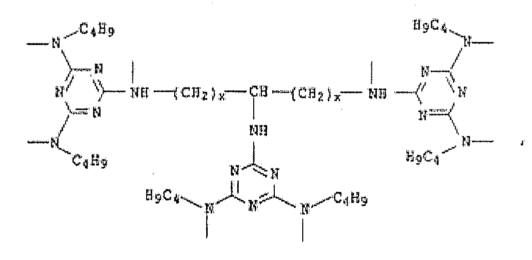 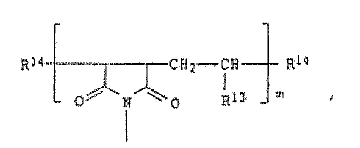 dans lequel
R¹⁰ signifie un alkyle en C₁-C₁₂ ou- (CH₂)_{z-}COOR⁹
R¹¹ signifie un hydrogène ou un alkyle en C₁-C₁₈,
R¹² signifie un alkyle en C₁-C₁₈, vinyle ou isopropényle,
R¹³ signifie un alkyle en C₅-C₂₂,
R¹⁴ signifie un hydrogène ou un radical organique, tel qu'il est produit habituellement lors de la polymérisation radicalaire des monomères de départ,
k signifie 0 ou 1
x signifie 1 à 12, et
n signifie un nombre pair m.

7. Composition de monomères selon les revendications 1 à 6, qui contient en plus un ou plusieurs composés nitroso ou nitro aromatiques.

8. Composition de monomères selon les revendications 1 à 6, laquelle contient en plus un ou plusieurs co-stabilisants du groupe des phénothiazines, quinones, hydroquinones et leurs éthers, hydroxylamines ou phénylènediamines.

9. Procédé d'inhibition de la polymérisation précoce de monomères selon la revendication 1, **caractérisé en ce qu'**on ajoute aux monomères selon la revendication 1 au moins un composé N-oxylique d'une amine secondaire, laquelle ne porte aucun atome d'hydrogène sur les atomes de C-α, et en une quantité efficace comme stabilisant.

10. Procédé selon la revendication 9, **caractérisé en ce qu'**on ajoute aux monomères au moins un composé tétraalkylpipéridine-N-oxylique de formule II selon les revendications 5 et 6.

11. Procédé selon les revendications 9 et 10, **caractérisé en ce que** le monomère à stabiliser provient du groupe des N-vinylformamide, N-vinylpyrrolidone et N-vinylcaprolactame.

12. Utilisation des composés de formule II selon les revendications 5 et 6 pour la stabilisation des monomères de formule I selon la revendication 2 contre une polymérisation précoce.
